# EUROPEAN PATENT APPLICATION

(11) **EP 0 672 458 A2**
(43) Date of publication of application: **20.09.1995**
(21) Application number: 95301393.5
(22) Date of filing: 03.03.1995
(51) Int. Cl.: B03C 1/02, G01N 33/50, G01N 33/569, G01N 21/05, G01N 35/00

(54) **Method and apparatus for magnetic cytometry**

(30) Priority: 04.03.1994 US 206125
(71) Applicant: THE CLEVELAND CLINIC FOUNDATION, Cleveland, OH 44195 (US)
(72) Inventor: Zborowski, Maciej, Bay Village, Ohio 44140 (US); Green, Ralph, Shaker Heights, Ohio 44120 (US)
(74) Representative: Hackett, Sean James

(57) **Abstract**

Magnetic cytometry in a bulk liquid entails directing the liquid across an interpolar magnetic gap so as to deposit magnetically susceptible cells adjacent the gap. A selected gas, e.g., air, is then directed across the deposited cells (preferably after rinsing) to drive off excess liquid. A suitable cytometry apparatus (10) includes a syringe pump (15) for forcing bulk liquid from a holding reservoir (28) into a flow chamber disposed perpendicularly across the interpolar gap of a magnet. A second syringe pump (15a), which contains the rinse liquid, is equipped with a holding reservoir filled only part-way with the bulk liquid; the remaining portion is filled with the selected gas, which is forced through the flow chamber immediately after the rinse liquid. Preparation of cells for cytometry entails binding monoclonal antibodies to antigens that characteristically appear on the surfaces of target cell populations, and magnetically labeling the antibodies with a magnetic agent, preferably ferritin, to render the cells magnetically susceptible.

## Description

### BACKGROUND OF THE INVENTION

### A. Field of the Invention

This invention relates to techniques for separating and sorting lymphocytes and/or other cells in a bulk flow. Sorting techniques of this type are useful in flow cytometry and other analyses of the structure, function, and/or pathology of cells.

### B. Description of the Related Art

Conventional cell-sorting techniques frequently exploit the differing protein compositions of cell surfaces. Such techniques find wide application in research and in diagnostic procedures relating to cancer and immunodeficiency diseases, as well as in other biomedical applications.

Cell sorting based on cell surface protein composition has been spurred by the rapid development in recent years of an understanding of cellular biology. A correlation has been established between a cell's functional differentiation and the protein composition of its surface. This correlation provides the basis for numerous immunological methods of cell identification and classification according to cell differentiation, such methods including immunofluorescence microscopy, immunocyto-chemistry, enzyme immunoassay, and flow cytometry.

Lymphocyte identification by flow cytometry entails separation of white blood cells from red blood cells, followed by differential counting of the lymphocyte subpopulations. A predetermined subpopulation of the lymphocytes, e.g., those indicative of a specific medical condition is identified by characteristic proteins appearing on that subpopulation's cell surfaces. These proteins function as immunogenic antigens, enabling the preparation of monoclonal antibodies ("Mabs") with specific affinities therefor. Such Mabs can be used to "label" cells expressing the antigens, thereby making them "observable" and facilitating more detailed microscopic and/or other analyses. For example, by coupling the Mab to a chemical species capable of producing a detectable signal, it is possible to measure target lymphocyte populations by counting the occurrences and the strengths of the signals.

Functionally distinct subpopulations of lymphocytes are ordinarily defined by more than one antigenic surface protein; these groups are referred to as clusters of differentiation ("CDs"). For example, all mature T-lymphocytes exhibit a cluster of proteins collectively denoted the "CD3" marker; "helper/inducer" T-cells are characterized by the presence of a "CD4" marker; cytotoxic/suppressor T-cells by a "CD8" marker; lymphocytes displaying natural-killer cell activity by a "CD16" marker; stem-cell activity by a "CD34" marker; and all lymphocytes by a "CD45" marker. At present, highly pure populations of Mab for identifying each of these markers, and other CD markers, are commercially available for human and other lymphocytes, as well as for other cells.

These Mabs can be used in lymphocyte identification by flow cytometry to diagnose various diseases. For example, cell-marker studies are used for detection of helper T-cell deficiency in patients with acquired immunodeficiency syndrome (AIDS) or AIDS-related complex (ARC). For these conditions, the relative as well as absolute count of cells with certain CD markers may be important in diagnosis; thus, a reduced proportion of the CD4 marker indicative of helper/inducer, peripheral blood lymphocytes, relative to the CD8 suppressor/cytotoxic cell marker, supports the diagnosis of AIDS. Congenital immunodefiency states, such as severe combined immunodeficiency syndrome (also called "SCIDS" or "bubble boy" syndrome) are indicated by depletion of the CD3 marker for peripheral lymphocytes. Cancer can also be diagnosed using flow cytometry; leukemia and lymphoma cell populations display a characteristic antigenic phenotype that can be detected and classified through differentiation along a particular hematopoietic lineage.

In addition, flow cytometry has a significant role in tissue typing for renal and bone marrow transplantation, and in predicting transplant rejection on the basis of the ratio of the CD4 marker to the CD8 marker.

Accordingly, it can be appreciated that lymphocyte identification by flow cytometry can play a significant role in research, diagnosis and treatment of disease. Flow cytometry is currently practiced using any of a number of different labeling techniques, the most important involving the use of fluorescent chemical markers.

In conventional fluorescent-activated cell sorting ("FACS"), cells are labeled with one or more fluorescent dyes, such as fluorescein, attached to antibodies specific for the cells of interest. A mixture of cells including the labeled cells is pumped through a sophisticated fluidics and optics system. The system includes a laser beam for illuminating the fluid, and a number of detectors for measuring the resulting scatter and intensities of light. The type of cells can be identified by cell size and granularity as measured by light scatter, and by high and low fluorescence intensities indicating the presence or absence of bound, labeled antibody.

Advantages of FACS include high sensitivity of fluorescent detectors, which provide the ability to identify small populations of cells. FACS can quantify multiple cell sub-populations simultaneously by using muliple, different-color fluorescein labels associated with different antibodies. FACS may, however, produce unreliable results and present potential diagnostic pitfalls, particularly for samples from solid tissues, because the cells must be contained in single-cell suspensions. There is a risk, therefore, that faulty cell isolation will result in erroneous fluorescence data. In addition, FACS is limited in use to sorting small cell populations.

The details of FACS procedures are described in Parks et al., "Flow Cytometry and Fluorescence Activated Cell Sorting (FACS)," in Handbook of Experimental Immunology in Four Volumes, Vol. 1 (1986).

Cells have also been analyzed using magnetically labeled markers. However, such materials have not heretofore been utilized for cytometric studies that yield quantitative measurements of cell populations and subpopulations. Magnetic cell analysis may, for example, be based on ferrography, which is a method of separating out particles in a liquid by passing the liquid through a magnetic field. The interaction between the magnetic field and magnetic dipole moments of the particles causes the particles to be deposited on a substrate for further analysis. The apparatus for carrying out ferrography is called an "analytical ferrograph."

Ferrography has been widely used for industrial applications. U.S. Patent No. 4,047,814, issued to Westcott, describes several industrial uses for ferrography, such as monitoring wear debris in oil or grease lubricants. Ferrography has also been proposed for biomedical applications, such as analyses of wear in natural and prosthetic human joints. More recently, a magnetic separation method and apparatus dedicated to cell analysis has been proposed. U.S. Patent No. 5,053,344, issued to Zborowski, the inventor named herein, and Malchesky describes many of the problems encountered when attempting to apply industrial-type ferrography to the area of biological fluid analysis. These include less-than-optimal fluid dynamics, inadequate magnetic field configurations relative to the samples, and the lack of adequate magnetizing agents. Moreover, magnetic cell manipulation requires cumbersome precautions when used to isolate biological samples containing transmittable diseases.

The '344 patent involves a magnetic cell deposition system useful for flow cytometry. It employs a magnet having pole members that form an interpolar gap therebetween with a relatively high magnetic flux density. The system passes a biological fluid in a "closed" flow chamber through the fringing magnetic field adjacent to the gap. The flow chamber has opposing, optically transparent slides mounted so as to define a generally planar fluid pathway. Fluid flow is established by a combination of gravitational and capillary action. Particles that are magnetically susceptible (either naturally or due to prior preparation) are separated from the balance of the biological fluid, and are deposited on the slide nearer to the magnetic pole members.

Magnetic cell deposition appears to be a viable alternative to FACS for lymphocyte population analysis and has many advantages thereover, including being simpler and less expensive to implement and operate. These advantages are due in part to the fact that cell deposition concentrates the cells of interest and thus does not require sophisticated fluidic or optical systems for subsequent study or counting of the cells.

However, as currently practiced, magnetic cell analysis presents certain drawbacks. Inaccurate results may be produced because of lytic decomposition or disintegration of marked cells in the bulk flow, which can occur due to osmotic pressure differences that develop across cell membranes as the cells are isolated. Existing techniques for magnetically marking cells often prove inadequate as well, limiting the obtainable resolution. And present magnetic separation systems cannot simultaneously sort multiple cell populations from a single sample.

### DESCRIPTION OF THE INVENTION

### A. Summary of the Invention

It is an object of the invention to provide a technique for minimizing or preventing cell lysis so as to improve accuracy and performance in magnetic cytometry.

It is another object of the invention to provide improved techniques for preparing biological liquids for magnetic cytometry, including reliable and accurate methods for marking cells with a magnetic agent to render them susceptible to magnetic cytometry.

Yet another object of the invention is to provide a magnetic cytometry system capable of simultaneous identification and quantification of multiple cell populations.

In a first aspect of the invention, magnetic cytometry of selected, magnetically susceptible cells in a bulk liquid entails, first, directing the liquid across an interpolar gap of a magnet; second, directing a rinse solution across the interpolar gap; and third, directing a selected gas, e.g., air, across the deposited cellular material so to quickly remove excess liquid, thereby minimizing lysis.

A suitable apparatus for magnetic cytometry includes a system of syringes, pumps and valves for forcing bulk liquid and rinsing solutions from holding reservoirs into a flow chamber disposed perpendicularly across the interpolar gap. Alternatively, squeeze tubes or other means can be employed instead of syringe pumps. In any case, the holding reservoir or tube containing the rinse solution is filled only part way with this liquid. The remaining portion of the reservoir contains, e.g., air, which is forced through the flow chamber immediately after the rinse liquid. This drives off the liquid without first permitting the buildup of dangerously high ion concentrations, which can provoke cell lysis.

Preferably, the flow chamber is oriented so that flow extends vertically downward across the interpolar gap. As a result of this orientation, the present invention, unlike prior magnetic deposition arrangements or ferrograph systems generally, clearly separates the effects of gravity and magnetic force. Accordingly, the contents of the holding reservoir move downwardly within the flow chamber due to pumping pressure, capillary action and gravity, and deposition of the selected cells is due only to the lateral magnetic forces.

In a second aspect of the invention, preparation of the cells for sorting entails the steps of identifying antigens on cell surfaces using Mabs as "tags," and then labeling tagged cells with a magnetic agent (preferably in a physically dissolved or colloidal state, a suitable example being ferritin). The magnetic labeling protocol includes attaching the Mabs to the cells, and then attaching the magnetic agent to the bound Mabs, thereby sensitizing the cells for magnetic cytometry. Alternatively, the Mabs may be sensitized prior to attachment to the cells of interest.

In yet another aspect of the invention, a magnetic cytometry technique in accordance with an alternative embodiment employs a flow unit having multiple parallel flow chambers of the same geometry, which cross the interpolar gap perpendicularly. Each flow chamber has its own input port at one end and outlet port at the other end. In order to perform simultaneous sorting of a bulk liquid, different cell subpopulations of the liquid are magnetically marked, and the bulk liquid is directed from multiple holding reservoirs into all of the input ports. Different labeling can be used to sort differently labeled cell subpopulations in the different flow chambers, or the same labeling can be used to sort cells with different magnetic susceptibilities.

This embodiment can be used with particular advantage where ratios of different subpopulations are required or sought, because it avoids many extraneous experimental variables (e.g., temperature- and time-dependent sample changes) that could influence results if the experiments were conducted serially, one after another. Alternatively, of course, each flow chamber can be connected to a different holding reservoir in parallel, so that multiple samples can be sorted simultaneously to save time and improve experimental efficiency.

Another embodiment of the invention provides a serpentine-shaped flow chamber that crosses and re-crosses the interpolar gap. Magnetically labeled cells are deposited at each pass of the flow chamber in accordance with the degrees of magnetic susceptibility of the cells.

### B. Brief Description of the Drawings

For a fuller understanding of the nature of the invention, reference should be had to the following detailed description taken in connection with the accompanying drawings, in which:
Fig. 1 is a perspective view of an analytical magnetic cell separator in accordance with the invention;
Fig. 2 is a front view of the capillary flow unit and magnetic structure of Fig. 1;
Fig. 3 is an exploded view of the capillary flow unit of Fig. 1;
Fig. 4 is a front view of the rinse-liquid holding reservoir of the syringe of Fig. 1;
Figs. 5A and 5B are block diagrams in flow-chart form of alternative methods of cell preparation for analytical magnetic cytometry using the apparatus of Fig. 1;
Fig. 6 is a diagramatic view of a cell structure after labeling for analytic cell sorting in accordance with the methods of Figs. 5A and 5B;
Fig. 7 is an exploded view of a capillary flow unit with parallel flow chambers in accordance with an alternative embodiment of the invention; and
Fig. 8 is an exploded view of a capillary flow unit with a serpentine-shaped flow chamber in accordance with yet another embodiment of the invention.

### C. Detailed Description of the Invention

### 1) Analytical Magnetic Cytometer

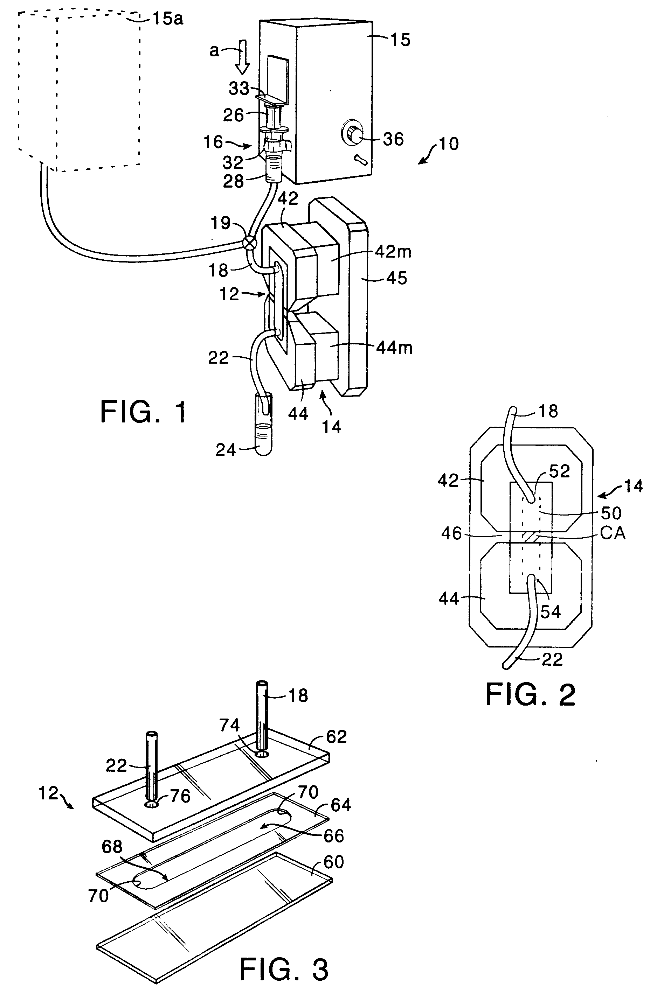

Fig. 1 shows an analytical magnetic cytometer ("AMC") 10 in accordance with a preferred embodiment of the invention. The AMC 10 includes a capillary flow unit 12 mounted within a magnetic field established by a magnet structure 14. AMC 10 also has a syringe pump 15 for directing a bulk liquid along an entirely closed (i.e., sealed) fluidic pathway from a syringe 16 through an inlet tube 18 that includes a three-way stopcock valve 19, to the capillary flow unit 12, and then through an outlet tube 22. The outlet tube 22 directs spent bulk liquid from the capillary flow unit 12 into a waste reservoir, e.g., a collection tube 24. The AMC 10 preferably includes a second syringe pump 15a, which directs a rinse liquid and a volume of gas, as described below, along an entirely closed fluidic pathway. The output of the latter pump joins inlet tube 18 at valve 19.

The syringe pumps 15 and 15a can be conventional units for automatically moving a plunger 26 of the syringe 16 into its holding reservoir 28, thereby ejecting the holding reservoir's contents into the inlet tube 18 at a regulated, predetermined flow rate. To that end, the pumps are each equipped with a collar arrangement 32 for clamping the holding reservoir 28, and a translatable flange member 32 that drives the plunger 26 linearly (in the direction of arrow "a") into the holding reservoir 28. The pumps can also have controls 36 for manual adjustment of the rate of ejection of the contents of the holding reservoir 28.

The magnetic structure 14 has first and second magnetically permeable pole members 42, 44 separated by an interpolar gap 46 that is best seen in Fig. 2, and a magnetically permeable support member 45. Sandwiched between the pole members and support member 45 are first and second permanent magnets 42m, 44m. The interpolar gap 46 is, e.g., 1.2 mm wide and 75 mm long. The pole members 42, 44 establish a magnetic flux density at the interpolar gap 46 that can range up to the saturation point of the pole pieces, generally on the order of 2 Tesla or so.

The capillary flow unit 12 includes a continuous, elongate flow chamber 50 that extends perpendicularly across the interpolar gap 46 approximately mid-way along the chamber's length. The chamber is disposed in a plane generally perpendicular to the opposed faces of pole members 42, 44, which define interpolar gap 46. The flow chamber 50 is thin, preferably only about 250 micrometers to about 500 micrometers in thickness in order to to keep flow volume to a minimum, thereby promoting a laminar flow profile and capillary effect. The inlet tube 18 is coupled to one end 52 of the chamber 50 (e.g., the top end) and the outlet tube 22 is coupled to the opposite end 54 (e.g., the bottom end) of the chamber 52.

It should be noted that the flow chamber 50 extends in the illustrated embodiment in a vertical direction. Vertical flow clearly separates the vertical force of gravity from the inward magnetic force, so that only the latter acts to retain magnetically tagged cells moving downwardly through flow chamber 50. These cells are transported from holding reservoir 28 (Fig. 1) by the pumping pressure, capillary action and gravity.

Fig. 3 shows an exploded view of the capillary flow unit 12. The unit 12 includes thin, co-planar, elongated top and bottom walls 60, 62, called "slides." The slides 60, 62 can be made, e.g., from glass or plexiglass and are preferably optically transparent. Interposed between the slides 60, 62 is a thin, elongated spacer 64. The spacer 64 includes an elongated channel 66, which, after the spacer 64 is sandwiched between the slides 60, 62, forms therein the flow chamber 50.

More specifically, the channel 66 includes straight side walls 68 and curved end walls 70. The channel 66 can be formed as a cut-out in an otherwise continuous sheet of spacer material. The spacer 64 is preferably made of a material that electrostatically adheres to the slides 60, 62, e.g., a silicon and elastomer material (such as the SILASTIC material marketed by Dow Corning, NY, NY), thereby self-sealing the periphery of the channel 66 after assembly with the slides.

The top slide 62 has an inlet port 74 that aligns after assembly with the top end 52 of the channel 66, and an outlet port 76 that aligns after assembly with the bottom end 54 of the channel 66. The inlet and outlet ports 74 and 76 are connected to respective inlet and outlet tubes 18 and 22. The inlet and outlet ports 74, 76 are separated by a distance selected to allow magnetic separation of the cells in the bulk liquid as it flows in the chamber 50 between the ports 74, 76.

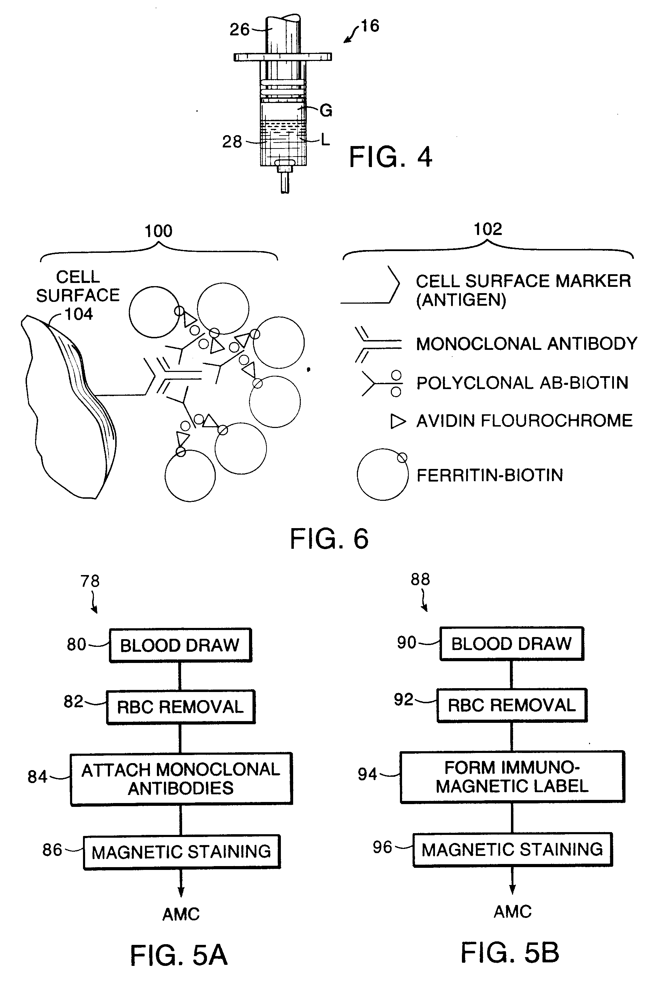

In accordance with the invention, as illustrated in Fig. 4, the syringe 16 associated with pump 15a is partially filled with a rinse liquid "L" (which may be, for example, a standard phosphate-buffered saline or electrolyte solution), and partially filled with a selected gas "G," e.g., air. The syringe 16 associated with pump 15 is filled with a bulk liquid containing magnetically labeled cells. During operation of the AMC 10, pump 15 causes syringe 16 to direct the bulk liquid in a thin laminar layer (i.e., a layer having a laminar velocity profile) into the flow chamber 50, and over a strongly inhomogeneous magnetic field localized adjacent to the gap 46 of the magnet 14. This field draws the labeled cells toward the inner slide 60, on which they are deposited in a collection area CA (Fig. 2) of the flow chamber 50 that overlies the gap 46. The unlabeled cells are carried away from the magnetic region and into collection tube 24. Subsequently, pump 15a is activated and valve 19 switched to allow flow from the syringe 16 associated with pump 15a to enter flow chamber 50 via inlet tube 18. Pump 15a causes syringe 16 to inject the rinse liquid L and thereafter the gas G across the deposited cellular material in the flow chamber 50. Gas G drives off excess bulk liquid, and the cells remain suspended in collection area CA. Rapid clearance of the liquid prevents cell lysis, which can result from the increasing ion concentrations that accompany evaporation of the liquid. The rate and efficiency of liquid clearance is assisted by the vertical orientation of flow chamber 50.

After completion of the air injection step, the labeled cells in the collection area may form a characteristic "signature" band based on relative magnetic susceptibilities, and in any case are available for conventional cytochemical staining and microscopic observation. These can be performed using additional syringes and syringe pumps that dispense the necessary chemicals into channel 66. For example, the collected cells can be analyzed by optical methods (e.g., transmitted-light attenuation, polarized-light analysis, scattered-light intensity analysis), fixing and histological staining (including immunohistological stains) followed by light densitometry analysis, and microscopic observation using either light or fluorescence microscopy. The collected cells can also be re-suspended for further processing by removing the slide 60 (Fig. 1) from the magnet 14 (Fig. 1), and gently washing the cells from the slide. Indeed, the collected cells can be alive, and cultures can be grown therefrom.

The advantages obtainable with the small, laminar flow rates facilitated by our design were confirmed in an experiment using freshly isolated human peripheral lymphocytes. The lymphocytes were stained with cationized horse spleen ferritin, which binds nonspecifically to negatively charged functional groups on the cell membrane surface. The ferritin molecules were clearly discernible on the cell surface in a transmission electron microscopy microphotograph of the lymphocyte stained with the cationized ferritin. After these cells were passed through the apparatus described above, cellular deposition on the slides, following air-fixation and staining, could be seen. Cell deposition in the collection area CA increased to 85% from 35% as the average linear velocity of bulk liquid flow decreased from 0.34 cm/sec to 0.07 cm/sec. Thus, the magnetic susceptibility of ferritin-stained lymphocytes proved effective in the environment of the present invention, and visible inspection confirmed well-preserved morphology of lymphocytes concentrated at the magnetic field focus.

### 2) Cell Preparation for AMC

The AMC 10 of Fig. 1 can be used to deposit particles or cells that are magnetically susceptible, whether naturally or due to prior preparation.

Fig. 5A shows a method 78 of cell preparation for analytical magnetic cytometry using the AMC 10. The method 78 begins with the drawing of blood, shown at block 80. At step 82, excess red blood cells ("RBCs") are removed from the blood, e.g., by centrifuging. Step 84 entails attaching Mabs to targeted lymphocytes. The same Mabs and controls for antibody specificity used for conventional FACS can be used in the AMC 10. Finally, at step 86, magnetic staining amplifies the magnetic susceptibility of the targeted lymphocytes. The magnetic staining causes the Mabs to be rendered magnetically susceptible, and thus affects the cells indirectly. Details of the magnetic labeling procedure are described below.

Fig. 5B shows an alternative method 88, which has blocks 90, 92 identical to blocks 80 and 82 of method 78. At step 94 the magnetic label is conjugated to the Mab, forming an immunomagnetic label. At step 96, this label is applied to the targeted lymphocytes.

Fig. 6 schematically illustrates the structure of a labeled cell 100, i.e., a cell that has been rendered magnetically susceptible for magnetic sorting in accordance with either method 78 of Fig. 5A or method 88 of Fig. 5B. The symbols used for representing the components of the labeled cell 100 are described in a legend 102 at the right of the drawing. The labeled cell has a cell surface 104 bearing a surface protein (i.e., antigen) to which a Mab having specific affinity therefor is attached. The Mab is itself labeled by specially prepared secondary antibodies having specific affinities for one or more regions of the bound Mab (and not for other cellular structures). These anti-Mab antibodies are themselves bound to a sandwich of fluorochrome and ferritin using an avidin-biotin system. The flurochrome can be used to permit a conventional FACS (not shown) to calibrate the AMC 10 and/or for post-collection cell analyses.

An exemplary labeling protocol for performing the method 78 of Fig. 5A is as follows: 1 ml of cell suspension in concentration of 4 x 10⁶ ml⁻¹, which has already been exposed to the primary Mab, is incubated with 1 to 10 µg of a selected secondary antibody; the secondary antibody has been biotinylated, and exhibits affinity for the bound Mab. The incubation time is typically 30 min of cooling, e.g., on ice, with occasional stirring. The cells are washed in a solution used as a medium in flow-cytometry analysis, containing FTA and heparin, but without serum (which may be a potential source of biotin and may inhibit avidin binding). 20-50 µg of a conjugate of avidin and fluorescein isothiocyanate (FITC) are added to the 1-ml cell suspension, now stained with the antibody. The solution is then incubated for 15 min on ice and washed in a flow wash solution.

Biotinylated ferritin, 20-50 µg, is then added to the cell suspension. Ferritin is an iron-storage protein naturally occuring in mammals, and preferably a soluble form is used. After incubation and subsequent washing in a hemagglutination buffer, the cells are ready for magnetic separation.

The ferritin can be biotinylated using a commercially available biotin N-hydroxysuccinimide derivative with an aminohexanoate spacer. The degree of biotinylation of the ferritin can be verified by passing the solution through a small glass wool column containing agarose-avidin and observing the change in light absorption over the course of reaction. A percent biotinylation higher than 95% can be routinely achieved using this approach.

### 3) Alternative Embodiments of Flow Unit

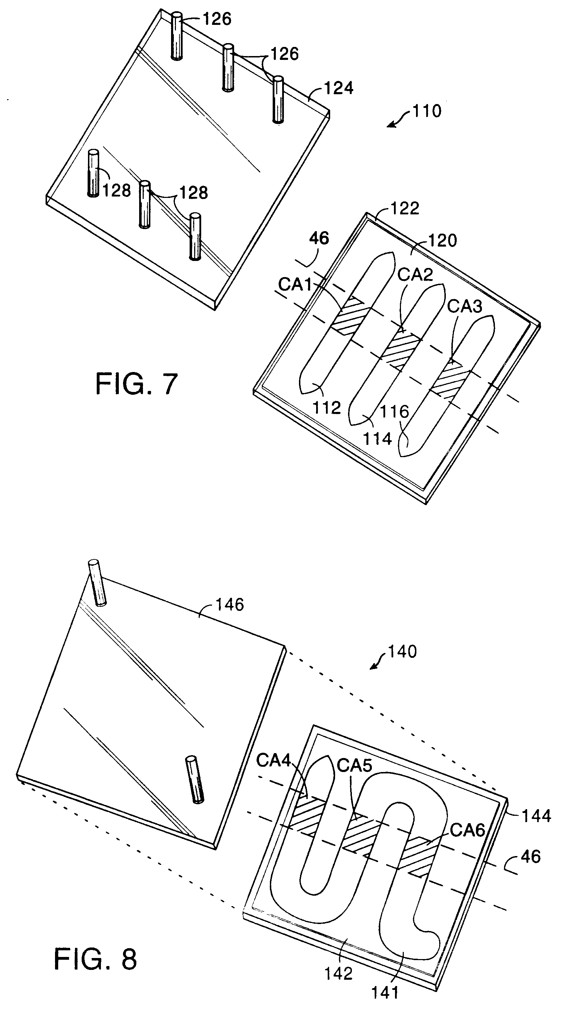

Fig. 7 shows a capillary flow unit 110 having a series of co-planar, parallel flow chambers 112, 114, 116 formed in a spacer 120 between slides 122, 124 in accordance with an alternative embodiment of the invention. Each of the flow chambers 112-116 preferably has the same geometry as the others, with side-by-side, and preferably equally sized rectangular flow collection areas CA1-CA3 disposed (after assembly) over the interpolar gap 46.

Each flow chamber 112-116 has its own input port 126 at one end and outlet port 128 at the other end. In order to perform simultaneous sorting of a bulk liquid, the liquid is divided into separate aliquots, and a different cell subpopulation magnetically marked in each. The aliquots are stored in separate holding reservoirs. Liquid is directed from each holding reservoir into a separate input port. Alternatively, different holding reservoirs can be used to hold separate, independent samples (i.e., from different sources), which can be sorted simultaneously to save time. (Sample runs may take about 20 minutes each, so three currently run samples can save 40 minutes.)

The capillary flow unit 110 can be used with particular advantage where ratios of different subpopulations are required, because simultaneous sorting of multiple cell types from the same sample avoids interference from extraneous experimental variables (e.g., temperature- and time-dependent concentration changes) that could influence results if the different cell types were sorted serially, one after another.

Fig. 8 shows a capillary flow unit 140 with a planar, serpentine flow chamber 141 formed in a spacer 142 between slides 144, 146 in accordance with yet another embodiment of the invention. The serpentine flow chamber 141 crosses and re-crosses the interpolar gap 46, thereby forming a plurality (e.g., three) of preferably rectangular collection areas CA4-CA6. In one version of this embodiment, the dimensions of the chamber 141 are identical at all of the collection areas CA4-CA6. Magnetically labeled cells are deposited at each pass of the flow chamber 141 in accordance with the degree of magnetic susceptibility of the labeled cells, since cells that were not drawn sufficiently close to slide 144 to be retained there during the first pass across interpolar gap 46 will be drawn more closely during successive passes. Laminar flow minimizes any tendency of the cells to drift during movement of the bulk liquid.

In a second version of this embodiment, the dimensions of flow chamber 142 are increased at successive crossings of interpolar gap 46, thereby incrementally decreasing the flow rate at each crossing. Smaller flow rates give the magnetic field more time to attract and retain, as they pass across interpolar gap 46, cells whose magnetic susceptibilities were insufficient to cause them to deposit during a previous pass.

Thus, it will be seen that a novel method and apparatus for magnetic cytometry has been described. The advantages of the invention set forth herein are but some that can be realized; others will be apparent to those skilled in the art. The terms and expressions that have been employed herein are terms of description and not of limitation. In the use of such terms and expressions, there is no intention of excluding any equivalents of the features shown and described. It is recognized that various modifications are possible within the scope of the invention as claimed.

## Claims

1. Apparatus for magnetic cytometry of selected magnetically susceptible cells contained a bulk liquid, said apparatus comprising:
a. a magnet having pole members defining an interpolar gap;
b. at least one flow chamber disposed adjacent to the interpolar gap;
c. means for forcing at least the bulk liquid into the flow chamber, thereby causing deposition of the selected cells within the flow chamber adjacent the interpolar gap; and
d. means for forcing a preselected gas across the deposited cells, thereby driving off any liquid.

2. The apparatus in accordance with claim 1 further comprising means for forcing a rinse liquid into the chamber after the bulk liquid but before the preselected gas.

3. The apparatus in accordance with claim 1 further comprising means for forcing a cytochemical stain into the chamber after the preselected gas.

4. The apparatus in accordance with claim 1 wherein the flow chamber is disposed perpendicularly to the interpolar gap.

5. The apparatus in accordance with claim 1, wherein the at least one flow chamber is oriented vertically and bulk fluid is directed downward across the interpolar gap.

6. Apparatus for magnetic cytometry of selected magnetically susceptible cells contained a bulk liquid, said apparatus comprising:
a. a magnet having pole members defining a horizontal interpolar gap;
b. at least one vertically oriented flow chamber disposed adjacent to and extending perpendicularly to the interpolar gap; and
c. means for forcing at least the bulk liquid into and downwardly through the flow chamber, thereby causing deposition of the selected cells within the flow chamber adjacent the interpolar gap.

7. The apparatus in accordance with claim 1 or 6, wherein the at least one flow chamber area has a volume profile selected to provide laminar flow.

8. The apparatus in accordance with claim 1 or 6, wherein the at least one flow chamber area has a thickness ranging from about 250 micrometers to about 500 micrometers.

9. The apparatus in accordance with claim 2, wherein the rinse liquid and gas forcing means comprises a syringe including a holding reservoir for supplying the rinse liquid and the selected gas, and a plunger translatable within the syringe for forcing the rinse liquid and the gas into the flow chamber.

10. The apparatus in accordance with claim 9, further including means for translating said plunger in said holding reservoir at a preselected rate.

11. The apparatus in accordance with claim 1 or 6, wherein the at least one flow chamber includes a serpentine-shaped portion that crosses and re-crosses the interpolar gap.

12. The apparatus in accordance with claim 11 , wherein the flow chamber has dimensions that are identical at each cross of the interpolar gap.

13. The apparatus in accordance with Claim 11, wherein the flow chamber has dimensions that differ at different crosses of the interpolar gap.

14. The apparatus in accordance with claim 1 or 6 comprising a plurality of identical flow chambers disposed adjacent to and extending perpendicularly to said interpolar gap.

15. The apparatus in accordance with claim 14, wherein said apparatus further comprises single supply means for supplying the bulk liquid into all input ports from a common reservoir.

16. The apparatus in accordance with claim 14, wherein said apparatus further comprises multiple supply means for supplying bulk liquid from a different reservoir into each input port.

17. A method for magnetic cytometry of selected magnetically susceptible cells contained a bulk liquid, the method comprising the steps of:
a. providing a flow chamber disposed adjacent to an interpolar magnetic gap;
b. forcing at least the bulk liquid into the flow chamber, thereby causing deposition of the selected cells within said flow chamber adjacent the interpolar gap; and
c. forcing a preselected gas across said deposited cells to drive off the bulk liquid.

18. The method in accordance with claim 17 further comprising the step of forcing a rinse liquid into the chamber after the bulk liquid but before the preselected gas.

19. The method in accordance with claim 17 further comprising means for forcing a cytochemical stain into the chamber after the preselected gas.

20. The method in accordance with claim 17 wherein the flow chamber is disposed perpendicularly to the interpolar gap.

21. The method in accordance with claim 17, wherein the forcing steps include directing the bulk liquid vertically across and in a plane parallel to first and second magnetic members between which the interpolar gap is formed.

22. The method in accordance with claim 17, further comprising the steps of rendering the selected cells magnetically susceptible by conjugating a magnetic agent to antibodies having specific affinity for antigens on the surfaces of the cells, and binding the antibodies to the antigens.

23. The method in accordance with claim 22 wherein the magnetic agent is conjugated to the antibodies before they are bound to the antigens.

24. The method in accordance with claim 22 wherein the magnetic agent is conjugated to the antibodies after they are bound to the antigens.

25. The method in accordance with claim 22, wherein the magnetic agent comprises ferritin.
